# EUROPEAN PATENT SPECIFICATION

(11) **EP 1 536 765 B1**
(45) Date of publication and mention of the grant of the patent: **31.10.2007**
(21) Application number: 03795439.3
(22) Date of filing: 12.09.2003
(51) Int. Cl.: A61K 8/895, A61K 8/97, A61K 8/89, A61Q 5/00, A61Q 19/00

(54) **AQUEOUS EMULSION COMPRISING AN ORGANOSILICON POLYMER**
WÄSSRIGE EMULSION MIT EINEM ORGANOSILICON-POLYMER
EMULSION AQUEUSE COMPRENANT UN POLYMERE ORGANOSILICIE

(30) Priority: 13.09.2002 JP 2002268949; 11.08.2003 JP 2003291014
(43) Date of publication of application: 08.06.2005
(73) Proprietor: Dow Corning Toray Co., Ltd., Tokyo (JP)
(72) Inventor: MORITA, Y., Dow Corning Toray Silicone Co., Ltd., Ichihara-shi, Chiba 299-0108 (JP); KOBAYASHI, K., Dow Corning Toray Silicone Co., Ltd, Ichihara-shi, Chiba 299-0108 (JP); MIZUTANI, Y., Dow Corning Toray Silicone Co., Ltd, Ichihara-shi, Chiba 299-0108 (JP); OZAKI, Masaru, Dow Corning Toray Silicone Co., Ltd, Ichihara-shi, Chiba 299-0108 (JP)
(74) Representative: Kyle, Diana
(86) International application number: PCT/JP2003/011747
(87) International publication number: WO 2004/024117

(56) References cited:
- EP-A- 0 640 658
- EP-A- 0 874 017
- PATENT ABSTRACTS OF JAPAN vol. 1999, no. 14, 22 December 1999 (1999-12-22) & JP 11 246769 A (DOW CORNING TORAY SILICONE CO LTD), 14 September 1999 (1999-09-14)

## Description

### Technical Field

The present invention relates to an aqueous emulsion comprising a linear organosilicon polymer, to a process for producing it, and to cosmetic materials incorporating said emulsion. In particular, it relates to an aqueous emulsion of an organosilicon polymer superior in dispersibility and adhesive properties, to a process for producing it, and to cosmetic materials capable of imparting superior characteristics such as smoothness and moist feel to the skin and hair.

### Background Art

Because organosilicon polymers whose main chain is composed of diorganosiloxane units and alkylene units possess acid resistance and alkali resistance superior to those of diorganopolysiloxanes whose main chain is composed of diorganosiloxane units alone, they have been known as useful alkali-resistant antifoaming agents (see Japanese Patent Application Publication (Kokai) No. Hei 7-60008; and equivalent EP 640658 and US 5531929). Furthermore, because they exhibit the same superior mold release properties and water repellency as the diorganopolysiloxanes, they have been investigated for possible use in various other fields. For example, they have been investigated for possible use in cosmetic material applications and coating applications.

Known methods used in the production of such organosilicon polymers include methods, in which diorganopolysiloxanes having silicon-bonded hydrogen atoms only at the two ends of the molecular chain and diolefins having vinyl groups only at the two ends of the molecular chain are addition-polymerized using hydrosilation reaction catalysts (see Japanese Patent Application Publication (Kokai) No. Hei 1-217040.), and methods, in which diorganopolysiloxanes having silicon-bonded hydrogen atoms only at the two ends of the molecular chain and diorganopolysiloxanes having silicon-bonded alkenyl groups only at the two ends of the molecular chain are addition-polymerized using hydrosilation reaction catalysts (see Japanese Patent Application Publication (Kokai) No. Hei 7-82379). US Patent No.6,013,682 describes a method of making a silicone in water emulsion comprising mixing materials comprising (I) a composition containing at least one polysiloxane, at least one organosilicon material that reacts with said polysiloxane by a chain extension reaction and a metal containing catalyst for said chain extension reaction, (II) at least one surfactant and (III) water to form a mixture; and emulsifying the mixture.

In addition, the present inventors have already proposed a process for producing organosilicon polymer emulsions, in which diorganopolysiloxanes having silicon-bonded hydrogen atoms only at the two ends of the molecular chain and diorganopolysiloxanes having silicon-bonded alkenyl groups only at the two ends of the molecular chain are addition-polymerized in an emulsified state (see Japanese Patent Application Publication (Kokai) No. Hei 11-246769).

However, despite the fact that the high-molecular organosilicon polymer emulsions obtained by the above-described methods were superior in lubricating properties, their dispersibility and stability when combined with other ingredients were insufficient; and, in addition, another defect they had consisted in poor adhesive properties with respect to various substrate materials. For this reason, their characteristic properties could not be fully revealed. For instance, when the high-molecular organosilicon polymers were combined with cosmetic materials, their lubricating properties, the moist feel, and other effects were difficult to obtain.

It is an object of the present invention to provide an aqueous emulsion comprising an organosilicon polymer possessing superior lubricating properties as well as excellent dispersibility and adhesive properties, a process for producing it, and cosmetic materials capable of imparting superior characteristics such as smoothness and moist feel to the skin and hair.

### Disclosure of Invention

The present invention relates to:
an aqueous emulsion comprising in the disperse phase a mixture consisting of: (A) a linear organosilicon polymer whose main chain is composed of diorganosiloxane units and alkylene units and (B) an oil that is liquid at room temperature and which does not contain hydrosilation reactive groups the weight ratio of component (A) and component (B) in said mixture being (A):(B) = 1:2 to 1:50).
The invention also relates to a process for producing an aqueous emulsion of a mixture consisting of (A) a linear organosilicon polymer whose main chain is composed of diorganosiloxane units and alkylene units and (B) an oil that is liquid at room temperature and that does not contain hydrosilation-reactive groups, in which a mixture of (a) a diorganopolysiloxane having silicon-bonded hydrogen atoms at the two ends of the molecular chain, (b) a diolefin or diorganopolysiloxane having silicon-bonded alkenyl groups at the two ends of the molecular chain, and (c) an oil liquid at room temperature that does not contain hydrosilation-reactive groups is emulsified in water, and, in this state, component (a) and component (b) are addition-polymerized using (d) a hydrosilation reaction catalyst, wherein the weight ratio of (a) + (b) : (B) = 1 : 2 to 1 : 50.
Furthermore, the present invention relates to cosmetic compositions comprising cosmetic materials and such aqueous emulsions.

### Brief Description of Drawings

Fig. 1 is a scanning microscope microphotograph of the hair surface treated in Practical Example 11 with the aqueous emulsion obtained in Practical Example 6.

Fig. 2 is a scanning microscope microphotograph of the hair surface treated in Comparative Example 9 with the aqueous emulsion obtained in Comparative Example 1.

### Best Mode of Invention

The organosilicon polymer of component (A) is composed of diorganosiloxane units and alkylene units. Normally, both ends of each alkylene unit are bonded to the silicon atoms of the adjacent diorganosiloxane units. The organosilicon polymer is preferably a linear or partially branched linear high-molecular compound that does not have cross-linked structures in the molecule. Normally, it is soluble in organic solvents such as toluene, etc. Specifically suggested are polymers made up of alternatingly arranged alkylene units represented by the formula: 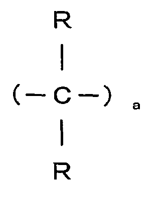 and diorganosiloxane units represented by the formula: 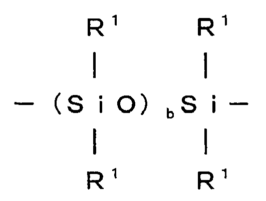 In the formulae, R stand for hydrogen atoms or monovalent organic groups, with monovalent hydrocarbon groups being representative of such monovalent organic groups. The monovalent hydrocarbon groups are exemplified by methyl, ethyl, propyl, butyl, pentyl, hexyl, and other alkyl groups, as well as by phenyl, benzyl, and other aryl groups. Hydrogen atoms are preferable. R¹ are monovalent organic groups, generally monovalent hydrocarbon groups other than alkenyl. They are specifically exemplified by alkyl groups, such as methyl, ethyl, propyl, butyl, pentyl, hexyl, heptyl, octyl, nonyl, and decyl; cycloalkyl groups, such as cyclopentyl and cyclohexyl; aryl groups, such as phenyl, tolyl, and xylyl; aralkyl groups, such as benzyl and phenetyl, and other unsubstituted monovalent hydrocarbon groups, halogenated alkyl groups, such as 3-chloropropyl and 3,3,3-trifluoropropyl, and other halogen-substituted monovalent hydrocarbon groups. Among them, methyl and phenyl are preferable. The subscript «a» is an integer of 2 or higher and the subscript «b» is an integer of 1 or higher. The subscripts may be identical or different. Preferably, the subscript «a» is 2~10 and the subscript «b» is 10 or higher, and even more preferably, the subscript «a» is 2-6 and the subscript «b» is 100 or higher. In addition, although the main chain of the organosilicon polymer of the present component is composed substantially of the above-mentioned alkylene units and diorganosiloxane units, arylene units may be included as part of it. There are no limitations on the number-average molecular weight of component (A). The molecular weight is at least 100,000, still more preferably, at least 200,000, and especially preferably, at least 300,000 up to 10,000,000 or more. Its viscosity at 25°C is preferably at least 100,000 mPa·s, more preferably, at least 1,000,000 mPa·s, and still more preferably, at least 100,000,000 mPa·s. Alkenyl groups and silicon-bonded hydrogen atoms originating from the raw material are present at the ends of the molecular chain of the organosilicon polymer. The two terminal groups may be identical or different. When addition polymerization is carried out using an excessive amount of component (a) relative to component (b), as described later, the terminal groups of the molecular chain are mostly silicon-bonded hydrogen atoms; when component (a) and component (b) are used in equivalent molar amounts, there are both silicon-bonded hydrogen atoms and alkenyl groups; and when an excessive amount of component (b) is used relative to component (a), the terminal groups are mostly alkenyl groups. Dimethylvinylsiloxy, dimethylallylsiloxy, dimethylhexenylsiloxy, and other dialkylalkenylsiloxy groups, as well as dimethylhydrogensiloxy are specifically suggested as the terminal siloxane units. In addition, dimethylhydroxysiloxy groups may be produced by the hydrolysis of the dimethylhydrogensiloxy groups. Furthermore, when a diorganopolysiloxane having a silicon-bonded hydrogen atom or a silicon-bonded alkenyl group at one end of the molecular chain and a trialkylsiloxy group at the other end of the molecular chain is used as an end-blocking agent during the synthesis of the present component, the resulting terminal siloxane units are trialkylsiloxy groups.

A non-crosslinkable silicone oil or an organic oil is suggested as Component (B), which is used to improve the dispersibility and adhesive properties of component (A). In addition, when component (A) is synthesized by means of a hydrosilation reaction, the component preferably does not contain alkenyl groups, SiH groups, and other functional groups that may exert influence on the hydrosilation reaction. Polyorganosiloxane oils with molecular structures such as linear, partially branched linear, cyclic, branched, etc. are preferable as the silicone oils, with linear or cyclic polyorganosiloxane oils being particularly preferable. Specifically suggested are dimethylpolysiloxanes having both ends of the molecular chain blocked by trimethylsiloxy groups, methylphenylpolysiloxanes having both ends of the molecular chain blocked by trimethylsiloxy groups, copolymers of methylphenylsiloxane and dimethylsiloxane having both ends of the molecular chain blocked by trimethylsiloxy groups, copolymers of methyl(3,3,3-trifluoropropyl)siloxane and dimethylsiloxane having both ends of the molecular chain blocked by trimethylsiloxy groups, cyclic dimethylsiloxane, cyclic methylphenylsiloxane, and glycidoxypropyl-containing polyorganosiloxanes. Isobutane, isopentane, neopentane, methylpentane, dimethylbutane, methylhexane, ethylpentane, dimethylpentane, trimethylbutane, methylheptane, dimethylhexane, trimethylpentane, methyloctane, methylnonane, and other isoparaffins; *n-*butane, *n*-pentane, *n*-hexane, *n*-heptane, *n*-octane, n-nonane, *n*-decane, *n*-pentadecane, *n-*octadecane, and other *n*-paraffins; hexyl laurate, isopropyl myristate, myristyl myristate, cetyl myristate, 2-octyldodecyl myristate, isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cacao oil, jojoba oil, sesame-seed oil, safflower oil, soybean oil, *tsubaki* (Japanese Camellia seed) oil, squalane, persic oil, castor oil, mink oil, cottonseed oil, coconut oil, egg yolk oil, beef tallow, pork tallow, polypropylene glycol monooleate, neopentyl glycol-2-ethylhexanoate, and other glycol ester oils; isostearic acid triglyceride, coconut oil fatty acid triglyceride, and other polyhydric alcohol ester oils; and polyoxyethylene lauryl ether, polyoxypropylene cetyl ether, and other polyoxyalkylene ether oils are suggested as the organic oils. Among them, polyorganosiloxane oils such as polydimethylsiloxane, etc., as well as isoparaffin oils are preferable. The viscosity of the oil component at 25°C is preferably not more than 100,000 mPa·s, more preferably, not more than 50,000 mPa·s, and still more preferably, from 0.1mPa·s up to 30,000 mPa·s.

The weight ratio of component (A) to component (B) is (A):(B) = 1:2 to 1:50, when the number average molecular weight of component (A) is 100,000 or more.

The mixture of component (A) and component (B) is a homogeneous mixture, in which component (A) and component (B) are dissolved without separation. In particular, when component (A) is of high viscosity, component (A) dissolves in component (B) and forms a homogeneous mixture. The consistency of the mixture at room temperature is either liquid or gummy. The mixture is soluble in solvents, the solvents being toluene, xylene, and other aromatic solvents; hexane, heptane, and other aliphatic solvents, as well as ketone solvents and ether solvents. The viscosity of the mixture at 25°C is preferably not more than 1,000,000 mPa·s, more preferably, not more 300,000 mPa·s, and still more preferably, from 1 mPa·s up to 100,000 mPa·s. The mixture of component (A) and component (B) can be retrieved from the emulsion of the present invention by removing water by air-drying, hot air drying, vacuum drying, drying under heating, and other means. The average particle size of the emulsion of the present invention is preferably in the range of from 0.01 to 500 µm, and more preferably, in the range of from 0.1 to 50 µm.

Although there are no limitations on the amount of the added water, preferably, it is in the range of from 10 to 2,000 parts by weight, and more preferably, in the range of from 20 to 1,000 parts by weight per 100 parts by weight of the total of component (A) and component (B).

To improve its stability, the aqueous emulsion of the present invention is preferably prepared using a surface active agent. Such agents are specifically exemplified by hexylbenzenesulfonic acid, octylbenzenesulfonic acid, decylbenzenesulfonic acid, dodecylbenzenesulfonic acid, cetylbenzenesulfonic acid, myristylbenzenesulfonic acid, their sodium salts, and other anionic surface active agents; octyltrimethylammonium hydroxide, dodecyltrimethylammonium hydroxide, hexamethyltrimethylammonium hydroxide, octyldimethylbenzylammonium hydroxide, decyldimethylbenzylammonium hydroxide, dioctadecyldimethylammonium hydroxide, tallow trimethylammonium hydroxide, coconut oil trimethylammonium hydroxide, and other cationic surface active agents; polyoxyalkylene alkyl ether, polyoxyalkylene alkyl phenol, polyoxyalkylene alkyl ester, polyoxyalkylene sorbitan ester, polyethylene glycol, polypropylene glycol, ethylene oxide adducts of diethylene glycol trimethylnonanol, and polyester nonionic surface active agents; as well as mixtures of two or more of the above-mentioned surface active agents. Although there are no limitations on the amount, in which they are added, the amount is preferably in the range of from 0.01 to 50 parts by weight, and more preferably, in the range of from 0.1 to 20 parts by weight, per 100 parts by weight of the total of component (A) and component (B).

The aqueous emulsion of the present invention can be prepared by a process, in which a mixture of (a) a diorganopolysiloxane having silicon-bonded hydrogen atoms at the two ends of the molecular chain, (b) a diolefin or diorganopolysiloxane having silicon-bonded alkenyl groups at the two ends of the molecular chain, and (c) an oil liquid at room temperature that does not contain hydrosilation-reactive groups is emulsified in water, and, in this state, component (a) and component (b) are addition-polymerized using (d) a hydrosilation reaction catalyst.

In component (a), the silicon-bonded hydrogen atoms are preferably only at the two ends of the molecular chain. The groups other than the silicon-bonded hydrogen atoms are exemplified by methyl, ethyl, propyl, butyl, pentyl, hexyl, heptyl, octyl, nonyl, decyl, and other alkyl groups; cyclopentyl, cyclohexyl, and other cycloalkyl groups; phenyl, tolyl, xylyl, and other aryl groups; benzyl, phenetyl, and other aralkyl groups; and 3-chloropropyl, 3,3,3-trifluoropropyl and other halogenated alkyl groups. Among them, methyl and phenyl are preferable. The molecular structure of the diorganopolysiloxane is substantially linear, but a portion of the molecular chain may be branched. There are no limitations on its viscosity at 25°C, but in order for it to be emulsified in water, it is preferably not more than 100,000 mPa·s, and more preferably, not more than 1,000 mPa·s. Component (a) is exemplified by dimethylpolysiloxane having both ends of the molecular chain blocked by dimethylhydrogensiloxy groups and by diorganopolysiloxanes obtained by substituting ethyl, phenyl, 3,3,3-trifluoropropyl, etc. for some of the methyl groups in the dimethylpolysiloxane.

Either one of the two compounds, diolefin or diorganopolysiloxane having silicon-bonded alkenyl groups at the two ends of the molecular chain, or both compounds, can be used as component (b). The diorganopolysiloxane preferably has silicon-bonded alkenyl groups only at the two ends of the molecular chain

In the diorganopolysiloxane having silicon-bonded alkenyl groups only at the two ends of the molecular chain, the alkenyl groups are exemplified by vinyl, allyl, butenyl, pentenyl, hexenyl, heptenyl, octenyl, nonenyl, and decenyl. Among them, vinyl and allyl are preferable. In addition, groups other than the silicon-bonded alkenyl groups are exemplified by methyl, ethyl, propyl, butyl, pentyl, hexyl, heptyl, octyl, nonyl, decyl, and other alkyl groups; cyclopentyl, cyclohexyl, and other cycloalkyl groups; phenyl, tolyl, xylyl, and other aryl groups; benzyl, phenetyl, and other aralkyl groups; and 3-chloropropyl, 3,3,3-trifluoropropyl and other halogenated alkyl groups. Among them, methyl and phenyl are preferable. The molecular structure of the diorganopolysiloxane is substantially linear, but a portion of the molecular chain may be branched. There are no limitations on its viscosity at 25°C, but in order for it to be emulsified in water, it is preferably not more than 100,000 mPa·s, and more preferably, not more than 10,000 mPa·s. Component (b) is exemplified by dimethylpolysiloxane having both ends of the molecular chain blocked by dimethylvinylsiloxy groups, diorganopolysiloxanes obtained by substituting ethyl, phenyl, 3,3,3-trifluoropropyl, etc. for some of the methyl groups in the dimethylpolysiloxane, and diorganopolysiloxanes obtained by substituting allyl, hexenyl, etc. for the vinyl groups of the diorganopolysiloxanes.

The diolefins preferably have vinyl groups at the two ends of the molecular chain and are exemplified by 1,3-butadiene, 1,4-pentadiene, 1,5-hexadiene, and 1,7-octadiene. Furthermore, they can be used in combination with divinylbenzene.

The amount, in which component (b) is added, is 0.2 to 800 parts by weight, per 100 parts by weight of component (a).

Compounds that do not contain the hydrosilation-reactive groups in the above-described component (B) are suggested as component (c). The amount, in which component (c) is added, is preferably in the range of from 0.1 to 5,000 parts by weight, and more preferably, 5 to 1,000 parts by weight per 100 parts by weight of the total of component (a) and component (b).

In order to emulsify the mixture of component (a) and component (b) in water, the components may be homogeneously blended together and the blend dispersed in water using publicly known agitating/mixing equipment, such as a Homomixer, a paddle mixer, a Henschel mixer, a homogenizer/disperser, a colloid mixer, a propeller agitator, a homogenizer, a continuous in-line type emulsifier, an ultrasonic emulsifier, or a vacuum kneader. At such time, it is preferable to use the above-mentioned surface active agents. Among them, nonionic surface active agents are preferable because they exert little influence on the addition polymerization reaction.

Platinum catalysts, rhodium catalysts, and palladium catalysts are suggested as component (d), which is a catalyst used for synthesizing component (A) by promoting the addition polymerization reaction of component (a) and component (b). Among them, platinum catalysts are preferable because they are capable of appreciably promoting addition polymerization. Such catalysts are specifically exemplified by microparticulate platinum, platinum supported on silica micropowder, platinum supported on activated charcoal, chloroplatinic acid, alcohol solutions of chloroplatinic acid, platinum-alkenylsiloxane complexes, platinum/olefin complexes, and platinum/carbonyl complexes, with platinum-alkenylsiloxane complexes being especially preferable. The allcenylsiloxanes in question are exemplified by 1,3-divinyl-1,1,3,3-tetramethyldisiloxane, 1,3,5,7-tetramethyl-1,3,5,7-tetravinylcyclotetrasiloxane, allcenylsiloxanes obtained by substituting ethyl, phenyl, etc. for some of the methyl groups of these alkenylsiloxanes, and alkenylsiloxanes obtained by substituting allyl, hexenyl, etc. for the vinyl groups of these alkenylsiloxanes. Among them, 1,3-divinyl-1,1,3,3-tetramethyldisiloxane is preferable because of the good complex stability it provides. In addition, in order to improve the stability of the platinum-alkenylsiloxane complexes, it is preferable to prepare liquid catalysts by adding 1,3-divinyl-1,1,3,3-tetramethyldisiloxane, 1,3-diallyl-1,1,3,3-tetramethyldisiloxane, 1,3-divinyl-1,3-dimethyl-1,3-diphenyldisiloxane, 1,3-divinyl-1,1,3,3-tetraphenyldisiloxane, 1,3,5,7-tetramethyl-1,3,5,7-tetravinylcyclotetrasiloxane and other alkenylsiloxanes, as well as dimethylsiloxane oligomers and other organosiloxane oligomers. Among them, alkenylsiloxanes are preferable. Emulsions obtained by emulsifying liquid catalysts containing such platinum-alkenylsiloxane complexes as the principal component in water are preferable for use as component (d). In the emulsions, the volume-average particle size of the liquid catalyst dispersed in liquid droplet form, in other words, its average particle size in the volumetric particle size distribution, is preferably not more than 1 *µ*m, more preferably, not more than 0.8 µm, and still more preferably, not more than 0.5 µm. In addition, in the volumetric particle size distribution of the liquid catalyst, the proportion of the catalyst with a particle size of 1 µm or less is preferably not less than 40 wt%. Methods used for the preparation of emulsions obtained by the emulsification of liquid catalysts containing such platinum-alkenylsiloxane complexes as the principal component in water are exemplified, for instance, by a method, in which a liquid catalyst containing a platinum-alkenylsiloxane complex as the principal component is dispersed in water using publicly known agitating/mixing equipment. At such time, in order to improve the stability of the emulsion, it is preferable to use the above-described surface active agents. In addition, there is another method, in which a dispersion of a liquid catalyst containing a platinum-alkenylsiloxane complex as the principal component in a surface active agent is prepared in advance and then added to the emulsion of the mixture of the above-described components (a), (b) and (c), thus dispersing it in water. The same surface active agents as the ones mentioned above are suggested as the surface active agents to be used, with nonionic surface active agents being preferable because they exert little influence on the hydrosilation reaction. The amount, in which the surface active agents are added, is preferably in the range of from 0.01 to 1,000 parts by weight per 100 parts by weight of the liquid catalyst containing a platinum-alkenylsiloxane complex as its principal component. There are no limitations on the amount, in which component (d) is added, but in order to efficiently promote the addition polymerization reaction, the amount is such as to provide 0.1 to 1,000 ppm, more preferably, such as to provide 0.1 to 500 ppm, and still more preferably, such as to provide 1 to 50 ppm of platinum metal by weight relative to the total weight of the above-described component (a) and component (b).

In the method of the present invention, after component (d) is added to an emulsion of the mixture of component (a), component (b), and component (c) and homogeneously blended therewith, the blend can be either left stand as is or heated in order to promote the addition polymerization reaction. The temperature, to which they are heated, is preferably not more than 100°C, and more preferably, not more than 70°C. In addition, in the process of addition polymerization, the molecular weight of the resultant organosilicon polymer can be adjusted by adding a diorganopolysiloxane having a silicon-bonded hydrogen atom or a silicon-bonded alkenyl group only at one of the end terminals of the molecular chain and branching can be introduced in the molecular structure of the resultant organosilicon polymer by adding an organopolysiloxane having a single silicon-bonded hydrogen atom or silicon-bonded alkenyl group in a side chain of the molecular chain. Alternatively component (d) can be blended with a mixture of component (a), component (b), and component (c) and then emulsified, leading to formation of a polymer emulsion by the process of US Patent No.6,013,682.

The above-described aqueous emulsion of the present invention is a stable emulsion of a homogeneous mixture of an organosilicon polymer and liquid oil. The emulsion is characterized by superior compounding stability and dispersibility in water and other components. Furthermore, since the emulsion of the present invention has excellent adhesive properties with respect to various substrate materials, it has the advantage of readily revealing the characteristics of the organosilicon polymer. The emulsion of the present invention is useful as an additive for cosmetics, coating materials, etc., various coating agents, mold release agents used for organic resin molding, organic resin modifiers, lubricating agents, etc. The substrate materials, for which the emulsion of the present invention is suitable, are exemplified by hair, skin, nails, metals, organic resins, inorganic resins, glass, ceramics, fillers, construction materials, fiber, paper, etc. Among them, the emulsion of the present invention is particularly suitable for skin cosmetics and hair cosmetics. In addition, the invention is characterized in that the production process of the present invention permits efficient preparation of emulsions with a small particle size containing a finely dispersed mixture of a high-viscosity organosilicon polymer and an oil.

Because the cosmetic materials of the present invention are combined with the above-described aqueous emulsion, they are characterized by having excellent dispersibility and adhesive properties with respect to the skin and hair and by imparting superior moist feel and smoothness to the skin and hair. The content of the aqueous emulsion in the cosmetic materials of the present invention is preferably in the range of from 0.1 to 80 wt%, and more preferably, in the range of from 0.5 to 50 wt%.

In addition to the above-described anionic surface active agents, nonionic surface active agents, pH-adjusting agents, antiseptics, anti-mold agents, rust preventives, etc., the various raw materials that can be compounded with the cosmetic materials of the present invention when the latter are used as skin cosmetics are exemplified by avocado oil, almond oil, olive oil, cacao butter, sesame oil, wheat germ oil, safflower oil, shea butter, turtle oil, tsubaki oil, persic oil, castor oil, grape seed oil, macadamia nut oil, mink oil, egg yolk oil, Japan wax, coconut oil, rose hip oil, hydrogenated oil, or similar oils or fats; *Hoplosterbus altanticus* oil, carnauba wax, candelilla wax, spermaceti wax, jojoba oil, montan wax, beeswax, lanolin, or similar waxes; liquid paraffin, Vaseline, paraffin, ceresin, microcrystalline wax, squalane, or similar hydrocarbons; lauric acid, myristic acid, palmitic acid, stearic acid, oleic acid, behenic acid, undecylenic acid, oxystearic acid, linolic acid, lanoleic acid, synthetic fatty acids, and other higher fatty acids; ethyl alcohol, isopropyl alcohol, lauryl alcohol, cetyl alcohol, cetostearyl alcohol, stearyl alcohol, oleyl alcohol, behenyl alcohol, lanolin alcohol, hydrogenated lanolin alcohol, hexyl decanol, octyl dodecanol, isostearyl alcohol, or similar alcohols; cholesterol, dihydrocholesterol, phytosterol, or similar sterols; ethyl linolate, isopropyl myristate, lanolin fatty acid isopropyl ester, hexyl laurate, myristyl myristate, cetyl myristate, octyldodecyl myristate, decyl oleate, octyldodecyl oleate, hexyldecyl dimethyloctanoate, cetyl isooctanoate, cetyl palmitate, glycerol trimyristate, glycerol tri(caprylate/caprate), propylene glycol dioleate, glycerol triisostearate, glycerol triisooctanoate, cetyl lactate, myristyl lactate, diisostearyl maleate, or similar fatty acid esters; glycerol, propylene glycol, 1,3-butylene glycol, polyethylene glycol, sodium *d*,1-pyrrolidonecarboxylate, sodium lactate, sorbitol, sodium hyaluronate, or similar humectants; cationic surface active agents; betaine-type, amino acid-type, imidazole-type, lecithin, and other amphoteric surface active agents; iron oxides and other colored pigments; zinc oxides, titanium oxides, zirconium oxides, or similar white pigments; mica, talk, sericite, or similar extender pigments; dimethylpolysiloxane, methylphenylpolysiloxane, octamethytetracyclosiloxane, decamethylcyclopentasiloxane, polyether-modified silicone oil, amino-modified silicone oil, or similar silicone oils; purified water; carbinone, carrageenan, alginic acid, gum arabic, traganth, tragacanth, pectin, starch, xanthan gum, polyvinyl alcohol, polyvinyl pyrrolidone, sodium polyacrylate, polyethylene glycol, or similar thickeners; silicone-acryl copolymer, silicone resin, acrylic polymers, or similar film-forming agents; and, moreover, UV absorbers, antibacterial agents, anti-inflammatory agents, antiperspirants, agents, fragrances, antioxidants, and propellants.
In addition, specific examples of products used as skin cosmetic materials include hand creams, skin creams, foundations, eye shadows, face cleansers, and body shampoos.

When the cosmetic raw materials of the present invention are used for the preparation of hair cosmetic materials, hair cosmetics exhibiting excellent adhesion to hair and capable of imparting a superior moist feel and smoothness can be obtained by adding film-forming agents, antifreeze agents, oily components, emulsifiers, wetting agents, anti-dandruff agents, chelating agents, UV absorbers, fragrances, coloring materials, and various other raw materials in addition to the aforementioned anionic surface active agents, non-ionic surface active agents, pH adjusters, antiseptics, anti-mildew agents, rust preventives, etc. The following are specific examples of film-forming agents: copolymers of silicone compounds and polymers of (meth)acrylic radical-polymerizable monomers, non-functional silicone resins and silicone resins modified with amino groups and organic groups containing fluorine groups, poly(N-methylpyrrolidone), and poly(N-acylallcyleneimine). There are no special restrictions with regard to the antifreeze agents, but in general these agents can be represented by such substances as ethanol, isopropyl alcohol, 1,3-butylene glycol, ethylene glycol, propylene glycol, and glycerol. Oily components may be those normally used in cosmetics. Some representative examples of such substances include microcrystalline wax, paraffin wax, spermaceti wax, beeswax, Japan wax, sugar-cane wax, and other waxes or their mixtures, liquid paraffin, α-olefin oligomers, squalane, squalene, or similar hydrocarbon oils or their mixtures, cetanol, stearyl alcohol, isostearyl alcohol, hardened castor oil derived alcohol, behenyl alcohol, lanolin alcohol, or similar linear or branched, saturated or unsaturated, unsubstituted or hydroxy-substituted higher alcohols or their mixtures, palmitic acid, myristic acid, oleic acid, stearic acid, hydroxystearic acid, isostearic acid, behenic acid, castor oil fatty acids, coconut oil fatty acids, beef tallow fatty acids and other linear or branched, saturated or unsaturated, unsubstituted or hydroxy-substituted higher fatty acids or their mixtures, olive oil, coconut oil, rapeseed oil, palm oil, castor oil, hardened castor oil, peanut oil, beef tallow, hydrogenated beef tallow, jojoba oil, hardened jojoba oil, monostearic acid glyceride, monooleic acid glyceride, dipalmitic acid glyceride, trimyristic acid glyceride, oleyl oleate, isostearyl isostearate, palmityl behenate, isopropyl palmitate, stearyl acetate, dihydroxystearic acid esters, or similar esters, linear, branched, or cyclic low molecular silicone oils, amino-modified silicone oils, fatty acid-modified silicone oils, alcohol-modified silicone oils, polyether-modified silicone oils, phosphoric acid (phosphate) group containing silicone oil, sulfuric acid (sulfate) group containing silicone oil, fluoro-modified alkyl-containing silicone oil, alkyl-modified silicone oil, epoxy-modified silicone oil, or similar silicone oils, high molecular weight silicones, and silicone resins that are soluble in solvents, liquid or in a green state at room temperature, or possess thermoplastic properties, or their mixtures. It is recommended to use the aforementioned silicones in the form of emulsions, with the emulsifiers comprising, e.g., glycerol monostearate, sorbitan monopalmitate, polyoxyethylene cetyl ether, polyoxyethylene stearic acid ester, polyoxyethylene sorbitane monolaurate, or other emulsifiers normally used for these purposes. Wetting agents may be represented by hexylene glycol, polyethylene glycol 600, sodium pyroglutamate, and glycerol. Anti-dandruff agents may be represented by sulfur, selenium sulfate, zinc pyridium-1-thiol-N-oxide, salicylic acid, 2,4,4'-trichloro-2'-hydroxydiphenyl ether, and 1-hydroxy-2-pyridine compounds. Antioxidants may comprise BHA, BHT, and γ-oryzanol. Chelating agents may comprise ethyl diamine tetraacetic acid, citric acid, ethane-1-hydroxy-1,1-diphosphonic acid, or their salts. UV absorbers can be represented by benzophenone derivatives such as 2-hydroxy-4-methoxybenzophenone, benzotriazole derivatives such as 2-(2'-hydroxy-5'-methylphenyl)benzotriazole, and cinnamic acid esters. Furthermore, the cosmetic materials of the invention can also be compounded with various other components, such as glycerin, propylene glycol, dipropylene glycol, 1,3-butylene glycol, or similar polyhydric alcohols, monoalkyltrimethyl ammonium salts, dialkyldimethyl ammonium salts, or similar quaternary ammonium salts, and, more specifically, stearyltrimethylammonium chloride, behenyltrimethylammonium chloride, distearyldimethylammonium chloride, dibehenyldimethylammonium chloride, or similar cationic surface active agents or amphoteric surface active agents, squalane, lanolin, perfluoro polyethers, cationic polymers or similar sensitization improvers, propylene glycol, glycerol, sorbitol, or similar humectants, methyl cellulose, carboxyvinyl polymer, hydroxyethyl cellulose, polyoxyethylene glycol distearate, ethanol or similar viscosity adjusters, pearlescent agents, fragrances, colorants, dyes, propellants, vitamins, hair nutrients, hormones and other pharmaceuticals, triclosan, triclocarban, or similar antibacterial agents, potassium glycyrrhizinate, tocopherol acetate, and other anti-inflammatory agents, zinc pyrithione and octopirox, or similar anti-dandruff agents, methylparaben and butylparaben, or similar antiseptics, propellants, and other components described in the Encyclopedia of Shampoo Ingredients (Miscelle Press, 1985). The following are examples of hair-cosmetic products to which the cosmetic raw material of the invention is applicable: shampoos, hair rinses, hair conditioners, hair treatments, setting lotions, blow-styling agents, hair sprays, styling foams, styling jels, hair liquids, hair tonics, hair creams, hair-growth stimulators, hair-nourishing preparations, and hair dye compositions.

### Examples

The present invention will now be described in greater detail with reference to practical examples. In the examples, all viscosity values to 25°C. The average diameter of emulsion particles, dispersibility in water, the viscosity of mixtures of organosilicon polymers and oil in the emulsion, and the number-average molecular weight of organosilicon polymers were measured in accordance with the following methods. Additionally, the molecular structures of the organosilicon polymers were determined by means of 1H-NMR spectral analysis and Infrared Spectral Analysis. Hydroxylation reaction catalysts were used in the form of an emulsion produced by emulsifying in water a liquid catalyst consisting of a 1,3-divinyl-1,1,3,3-tetramethyldisiloxane solution of a platinum-1,3-divinyl-1,1,3,3-tetramethyldisiloxane complex (with a platinum metal concentration of 0.04 wt%). The volume-average particle size of the catalyst, measured by laser scattering using a Coulter N4 submicron particle sizer (from Coulter Electronics Co., Ltd.), was 0.05 µm, and the content (vol.%) of catalyst particles with dimensions of 1 µm or less was 95 wt%
- Average Size of Emulsion Particles
   The average size of emulsion particles (i.e., the average size of particles in a mixture of an organosilicon polymer and oil) was measured based on the median diameter (the particle size corresponding to 50% of the cumulative distribution) determined using a laser diffraction type particle size distribution analyzer [Model LA-500 from Horiba Ltd.].
- Dispersibility of Emulsion in Water
   The emulsion was dissolved in water so as to bring the concentration of the mixture of organosilicon polymer and oil to 0.2 wt%, and its dispersion stability (degree of separation) was examined 24 hours later. The symbol "X" means that particles of oil floated on the surface, the symbol "Δ" means a little oil floating on the surface was found, and the symbol "○" means no oil floating on the surface was found.
- Mixture viscosity and appearance of Organosilicon Polymer and Oil
   A mixture of organosilicon polymer and oil was prepared by drying the emulsion for 1 week in an air stream at room temperature and confirming the removal of moisture based on the decrease in weight. The viscosity of the mixture was measured using a single-cylinder rotary viscometer (Model Bismetron VG-DA from Shibaura Systems Co., Ltd.). The appearance of the mixture was also observed.
- Number-Average Molecular Weight of Organosilicon Polymer
   A mixture of organosilicon polymer and oil was prepared by drying the emulsion for 1 week in an air stream at room temperature and confirming the removal of moisture based on the decrease in weight. The mixture was dissolved in toluene, and a number-average molecular weight recalculated to polystyrene was determined by gel permeation chromatography using the top peaks.

### [Practical Example 1]

A uniform mixture was prepared from 19.6 parts by weight of a dimethylpolysiloxane with a viscosity of 55000 mPa·s having both ends of the molecular chain blocked by dimethylvinylsiloxy groups (vinyl group content: 0.088 wt%), 0.4 parts by weight of a 10 mPa·s dimethylpolysiloxane having both ends of the molecular chain blocked by dimethylhydrogensiloxy groups (content of silicon-bonded hydrogen atoms: 0.16 wt%), and 80 parts by weight of a 10 mPa·s dimethylpolysiloxane having both ends of the molecular chain blocked by trimethylsiloxy groups. The viscosity of the mixture was 59 mPa·s. The mixture was then combined and mixed with 3.4 parts by weight of a mixture of a secondary tetradecyl ether and a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the dimethylpolysiloxane having both ends of the molecular chain blocked by dimethylhydrogensiloxy groups and the dimethylpolysiloxane having both ends of the molecular chain blocked by dimethylvinylsiloxy groups which were contained in the emulsion. The emulsion was subjected to addition polymerization by allowing it to stand for 1 day at room temperature. The final emulsion comprised a mixture of an organosilicon polymer of the following formula: 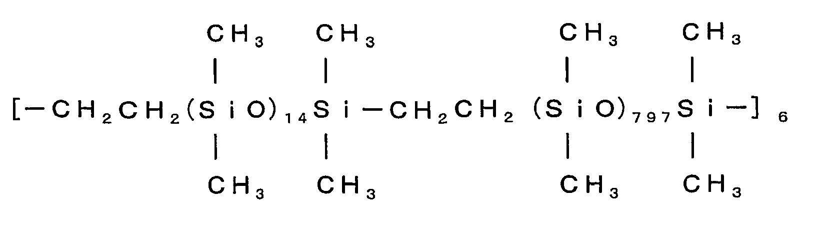 with a dimethylpolysiloxane having both ends of the molecular chain blocked by trimethylsiloxy groups, the mixture being emulsified and dispersed in water. The emulsion was examined to determine the average size of particles, dispersibility in water, mixture viscosity and appearance, and the number-average molecular weight of the organosilicon polymer. The results are shown in Table 1.

### [Practical Example 2]

A uniform mixture was prepared from 19.6 parts by weight of a 55000 mPa·s dimethylpolysiloxane having both ends of the molecular chain blocked by dimethylvinylsiloxy groups (vinyl group content: 0.088 wt%), 0.4 parts by weight of a 10 mPa·s dimethylpolysiloxane having both ends of the molecular chain blocked by dimethylhydrogensiloxy groups (content of silicon-bonded hydrogen atoms: 0.16 wt%), and 80 parts by weight of a 100 mPa·s dimethylpolysiloxane having both ends of the molecular chain blocked by trimethylsiloxy groups. The viscosity of the mixture was 407 mPa·s. The mixture was then combined and mixed with 3.4 parts by weight of a mixture of a secondary tetradecyl ether with a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the dimethylpolysiloxane having both ends of the molecular chain blocked by dimethylhydrogensiloxy groups and the dimethylpolysiloxane having both ends of the molecular chain blocked by dimethylvinylsiloxy groups which were contained in the emulsion. The emulsion was subjected to addition polymerization by allowing it to stand for 1 day at room temperature. The final emulsion comprised a mixture of the organosilicon polymer of the following formula: 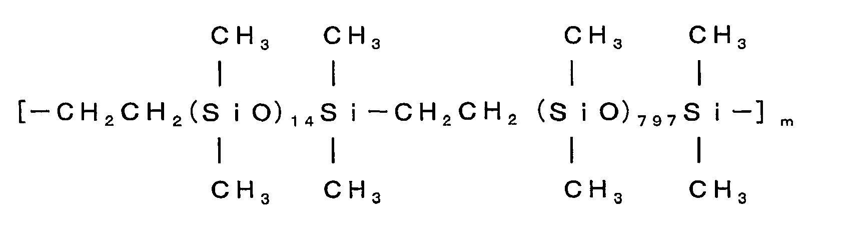 (where the subscript m is between 6 and 7) and the dimethylpolysiloxane having both ends of the molecular chain blocked by trimethylsiloxy groups, the mixture being emulsified and dispersed in water. The emulsion was examined to determine the average size of particles, dispersibility in water, mixture viscosity and appearance, and the number-average molecular weight of the organosilicon polymer. The results are shown in Table 1.

### [Practical Example 3]

A uniform mixture was prepared from 19.6 parts by weight of a 55000 mPa·s dimethylpolysiloxane having both ends of the molecular chain blocked by dimethylvinylsiloxy groups (vinyl group content: 0.088 wt%), 0.4 parts by weight of a 10 mPa·s dimethylpolysiloxane having both ends of the molecular chain blocked by dimethylhydrogensiloxy groups (content of silicon-bonded hydrogen atoms: 0.16 wt%), and 80 parts by weight of an isoparaffin oil ("Isozole 400K" from Nippon Petrochemicals Co., Ltd.). The mixture was then combined and mixed with 3.4 parts by weight of a mixture of a secondary tetradecyl ether with a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the diorganopolysiloxane which were contained in the emulsion. The emulsion was subjected to addition polymerization by allowing it to stand for 1 day at room temperature. The final emulsion comprised a mixture of the organosilicon polymer of the following formula: 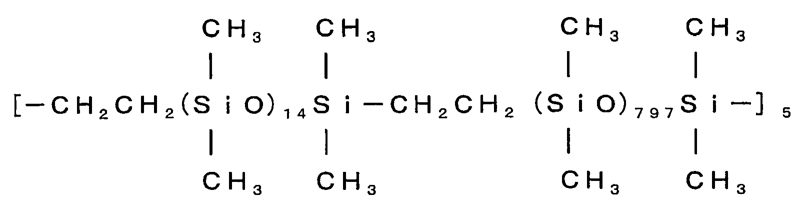 with isoparaffin oil, the mixture being emulsified and dispersed in water. The emulsion was examined to determine the average size of particles, dispersibility in water, mixture viscosity and appearance, and the number-average molecular weight of the organosilicon polymer. The results are shown in Table 1.

### [Practical Example 4]

A uniform mixture was prepared from 1.24 parts by weight of a 1,5-hexadiene, 18.76 parts by weight of a 10 mPa·s dimethylpolysiloxane having both ends of the molecular chain blocked by dimethylhydrogensiloxy groups (content of silicon-bonded hydrogen atoms: 0.16 wt%), and 80 parts by weight of a 10 mPa·s dimethylpolysiloxane having both ends of the molecular chain blocked by trimethylsiloxy groups. The mixture was then combined and mixed with 3.4 parts by weight of a mixture of a secondary tetradecyl ether with a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the dimethylpolysiloxane having both ends of the molecular chain blocked by dimethylliydrogensiloxy groups and 1,5-hexadiene which were contained in the emulsion. The emulsion was subjected to addition polymerization by allowing it to stand for 1 day at room temperature. The final emulsion comprised a mixture of the organosilicon polymer of the following formula: 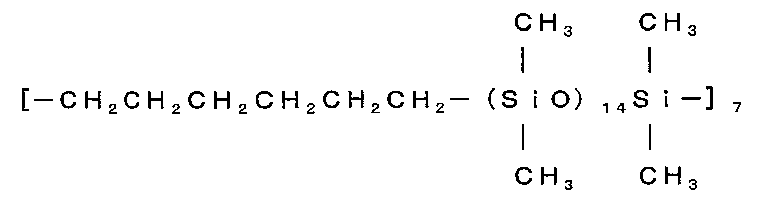 with dimethylpolysiloxane having both ends of the molecular chain blocked by trimethylsiloxy groups, the mixture being emulsified and dispersed in water. The emulsion was examined to determine the average size of particles, dispersibility in water, mixture viscosity and appearance, and the number-average molecular weight of the organosilicon polymer. The results are shown in Table 1.

### [Comparative Example 1]

A mixture was prepared from 9S parts by weight of a 55000 mPa·s dimethylpolysiloxane having both ends of the molecular chain blocked by dimethylvinylsiloxy groups (vinyl group content: 0.088 wt%) and 2 parts by weight of 10 mPa·s dimethylpolysiloxane having both ends of the molecular chain blocked by dimethylhydrogensiloxy groups (content of silicon-bonded hydrogen atoms: 0.16 wt%). The mixture was then combined and mixed with 3.4 parts by weight of a mixture of a secondary tetradecyl ether with a secondary dodecyl ether of ethylene oxide (12 moles added) (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl- 1,1,3,3-tetramethyldisiloxane complex (with a 0.04 wt% platinum metal concentration), so that the quantity of the platinum metal in weight terms was brought to 5 ppm of platinum metal relative to the total weight of the diorganosiloxane in the emulsion. The emulsion was subjected to addition polymerization by allowing it to stand for 1 day at room temperature. The final emulsion comprised an organosilicon polymer of the following formula: 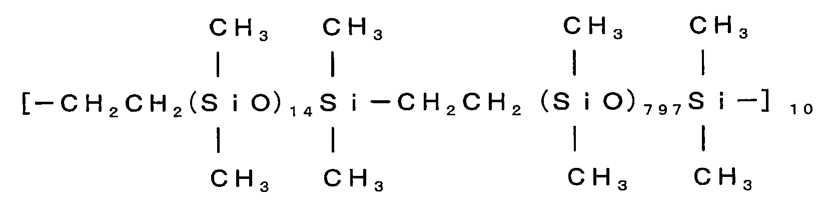 The emulsion was examined to determine the average size of particles, dispersibility in water, and the number-average molecular weight of the organosilicon polymer. The results are shown in Table 1. The viscosity of the obtained emulsion could not be measured by the aforementioned rotary viscometer and was determined using an ARES-type viscoelastomer (from Rheometric Scientific Co., Ltd.). The value of viscosity measured by this instrument at room temperature and under conditions shown below was 1,135 × 10⁵ mPa·s.
- Measurement Conditions:
   25-mm parallel plates; 1.0 mm gaps.
   Strain: 10%
   Oscillation frequency: 0.01 Hz

### [Comparative Example 2]

A mixture was prepared from 98 parts by weight of a 55000 mPa·s dimethylpolysiloxane having both ends of the molecular chain blocked by dimethylvinylsiloxy groups (vinyl group content: 0.088 wt%) and 2 parts by weight of 10 mPa·s dimethylpolysiloxane having both ends of the molecular chain blocked by dimethylhydrogensiloxy groups (content of silicon-bonded hydrogen atoms: 0.16 wt%). The mixture was then combined and mixed with a catalyst in the form of a platinum-1,3-divinyl-1,1,3,3-tetramethyldisiloxane complex added in such an amount that the content of the platinum metal in weight terms was 5 ppm relative to the total weight of the diorganosiloxane. The mixture was subjected to addition polymerization by allowing it to stand for 1 day at room temperature. The obtained product comprised a gum-like organosilicon polymer having a number-average molecular weight of 440,000 and expressed by the following formula: 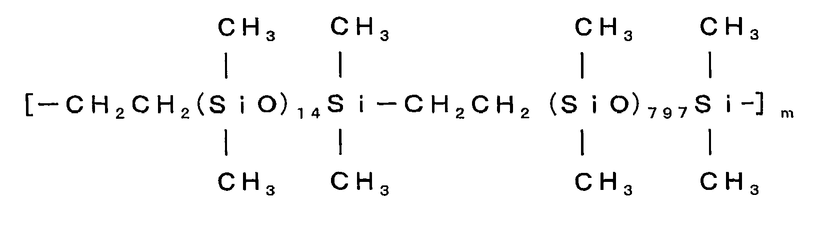 (where the subscript m is 7 or 8).
100g of the obtained organosilicon polymer and 80g of a 10 mPa·s dimethylpolysiloxane having both ends of the molecular chain blocked by trimethylsiloxy groups were placed in a 225-mL flask and mixed for 8 hours in a paint shaker. However, the obtained gum-like organosilicon could not be dissolved in dimethylpolysiloxane oil, and therefore a uniform mixture could not be obtained.

### [Comparative Example 3]

80g of a 10 mPa·s dimethylpolysiloxane having both ends of the molecular chain blocked by trimethylsiloxy groups were added to 1g of the gum-like organosilicon polymer with a number-average molecular weight of 440,000 obtained in Comparative Example 2.
100 parts of the obtained mixture were then combined and mixed with 3.4 parts by weight of a mixture of a secondary tetradecyl ether with a secondary dodecyl ether of ethylene oxide (12 moles added) (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was examined to determine the average size of particles, dispersibility in water, mixture viscosity and appearance, and the number-average molecular weight of the organosilicon polymer. The results are shown in Table 1.

### [Comparative Example 4]

100 parts by weight of a 100 mPa·s dimethylpolysiloxane having both ends of the molecular chain blocked by trimethylsiloxy groups were mixed with 3.4 parts by weight of a mixture of a secondary tetradecyl ether with a secondary dodecyl ether of ethylene oxide (12 moles added) (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, and an emulsion was obtained. The emulsion was examined to determine the average size of particles, and dispersibility in water. The results are shown in Table 1. The viscosity and the number-average molecular weight of the dimethylpolysiloxane which were contained in the emulsion were measured by the same methods as above. The viscosity was 100 mPa·s, and the number-average molecular weight was 7,000.

### [Practical Example 5]

A uniform mixture was prepared from 19.6 parts by weight of a 55000 mPa·s dimethylpolysiloxane having both ends of the molecular chain blocked by dimethylvinylsiloxy groups (vinyl group content: 0.088 wt%), 0.4 parts by weight of a 10 mPa·s dimethylpolysiloxane having both ends of the molecular chain blocked by dimethylhydrogensiloxy groups (content of silicon-bonded hydrogen atoms: 0.16 wt%), and 80 parts by weight of decamethylcyclopentasiloxane (with a viscosity of 4 mPa·s). The viscosity of the mixture was 33 mPa·s. The mixture was then combined and mixed with 3.4 parts by weight of a mixture of a secondary tetradecyl ether with a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the dimethylpolysiloxane having both ends of the molecular chain blocked by dimethylhydrogensiloxy groups and the dimethylpolysiloxane having both ends of the molecular chain blocked by dimethylvinylsiloxy groups which were contained in the emulsion. The emulsion was subjected to addition polymerization by allowing it to stand for 1 day at room temperature. The final emulsion comprised a mixture of the organosilicon polymer of the following formula: 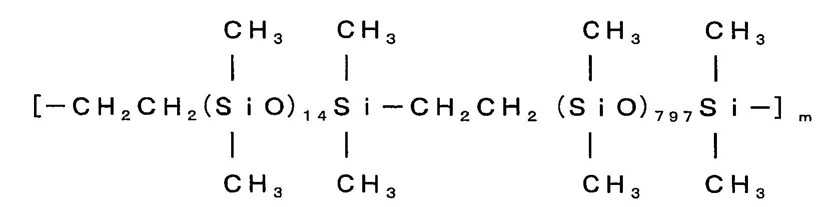 (where the subscript m is between 5 and 6) and the decamethylcyclopentasiloxane, the mixture being emulsified and dispersed in water. The emulsion was examined to determine the average size of particles, dispersibility in water, mixture viscosity and appearance, and the number-average molecular weight of the organosilicon polymer. The results are shown in Table 1.

### [Practical Example 6]

A uniform mixture was prepared from 19.6 parts by weight of a 55000 mPa·s dimethylpolysiloxane having both ends of the molecular chain blocked by dimethylvinylsiloxy groups (vinyl group content: 0.088 wt%), 0.4 parts by weight of a 10 mPa·s dimethylpolysiloxane having both ends of the molecular chain blocked by dimethylhydrogensiloxy groups (content of silicon-bonded hydrogen atoms: 0.16 wt%), and 20 parts by weight of a 30000 mPa·s dimethylpolysiloxane having both ends of the molecular chain blocked by trimethylsiloxy groups. Viscosity of the mixture was 33000 mPa·s. The mixture was then combined and mixed with 3.4 parts by weight of a mixture of a secondary tetradecyl ether with a secondary dodecyl ether of (12 moles) of ethylene oxide (the mixture contained 43 wt% of dodecyl groups, 57 wt% of tetradecyl groups, and had an HLB of 14.5), 4.7 parts by weight of a mixture of a secondary tetradecyl ether with a secondary dodecyl ether of (3 moles) of ethylene oxide (the mixture contained 43 wt% of dodecyl groups, 57 wt% of tetradecyl groups, and had an HLB of 8.0), and 10 parts by weight of water. After mixing the components, 80 parts by weight of water were added, the mixture was emulsified, and an emulsion was obtained. The emulsion was combined and homogeneously mixed with a catalyst in the form of a liquid catalytic emulsion of a platinum-1,3-divinyl-1,1,3,3-tetramethyldisiloxane complex (with a 0.04 wt% platinum metal concentration), so that the quantity of the platinum metal in weight terms was brought to 5 ppm of platinum metal relative to the total weight of the dimethylpolysiloxane having both ends of the molecular chain blocked by dimethylhydrogensiloxy groups and the dimethylpolysiloxane having both ends of the molecular chain blocked by dimethylvinylsiloxy groups which were contained in the emulsion. The emulsion was subjected to addition polymerization by allowing it to stand for 1 day at room temperature. The final emulsion comprised a mixture of the organosilicon polymer of the following formula: 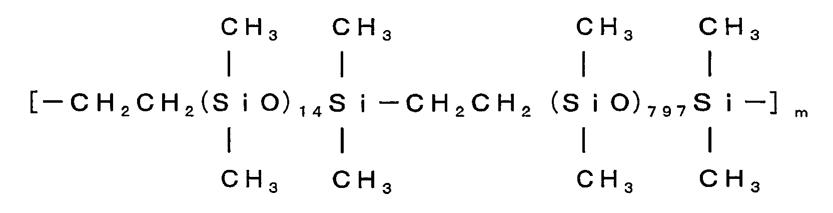 (where the subscript m is between 6 and 7) and the 30000 mPa·s dimethylpolysiloxane having both ends of the molecular chain blocked by trimethylsiloxy groups, the mixture being emulsified and dispersed in water. The emulsion was examined to determine the average size of particles, dispersibility in water, mixture viscosity and appearance, and the number-average molecular weight of the organosilicon polymer. The results are shown in Table 1.

**Table 1**

| Example No. | Average Particle Size (µm) | Dispersibility in water | Viscosity of Mixture (mPa·s) | Appearance of Mixture | Number-Average Molecular Weight of Organosilicon Polymer |
|---|---|---|---|---|---|
| Pr. Ex. 1 | 0.5 | ○ | 16,000 | Uniform | 380,000 |
| Pr. Ex. 2 | 0.6 | ○ | 42,000 | Uniform | 400,000 |
| Pr. Ex. 3 | 0.5 | ○ | 1,200 | Uniform | 310,000 |
| Pr. Ex. 4 | 0.5 | ○ | 250 | Uniform | 8,800 |
| Pr. Ex. 5 | 0.5 | ○ | 6,000 | Uniform | 340,000 |
| Pr. Ex. 6 | 2.9 | ○ | 900,000 | Uniform | 450,000 |
| Comp. Ex. 1 | 1.2 | ○ | No mixture | - | 620,000 |
| Comp. Ex.3 | 2.8 | × | 14 | Uniform | 440000 |
| Copm. Ex.4 | 0.5 | Δ | No mixture | - | No organosilicon polymer |

### [Practical Example 7]

2 drops of each emulsion obtained in Practical Examples 1 to 4 were placed on the back of the hand and rubbed in by a finger. The smoothness and tackiness to touch after evaporation of the moisture component were evaluated using the criteria given below. The results of the evaluation are shown in Table 2.
- Smoothness
   o: Low resistance to finger movement, sensation of smoothness.
   Δ: Sensation of smoothness, albeit rather "heavy" to the touch.
   x: No smoothness, strain sensation.
- Tackiness
   O : No sensation of resistance when the finger is slowly separated from the back of the hand.
   Δ: Sensation of slight tackiness when the finger is slowly separated from the back of the hand.
   × : Noticeable sensation of tackiness when the finger is slowly separated from the back of the hand.

### [Comparative Example 5]

The emulsions obtained Comparative Examples 1, 3, and 4 were evaluated for smoothness and tackiness by methods described in Practical Example 7. The results are shown in Table 2.

### [Practical Example 8]

The emulsions obtained in Practical Examples 1 to 4 were diluted with water until the concentration of the mixture the organosilicon polymer and oil reached 0.2 wt%. Furthermore, a 5g lock of hair was washed with an aqueous solution of 1 wt% sodium polyoxylaurylsulfate, the water was drained, and the hair was dried for 12 hours at 25°C. The treated hair was then dipped for 30 sec. to a 500 g aqueous solution of the aforementioned emulsion. After draining the water, the remaining water was wiped off with a kimtowel wiper, and weighed, and the coefficient of adhesion (%) was calculated.
The hair treated by immersion into the aforementioned solution was evaluated with regard to tactile sensation by a panel of 5 people. The symbol "o" corresponds to the case when three or more people evaluated the sensation as smooth and moist, the symbol "×" corresponds to the case when three or more people considers the hair either not smooth or not moist, and the symbol "Δ" corresponds to the case was neither "o" nor "×". The results are shown in Table 2.

### [Comparative Example 6]

The emulsions obtained in Comparative Examples 1, 3, and 4 were used for treating a hair lock by the same method as in Practical Example 8, whereupon the treated hair was evaluated by determining the weight of the adhered substance, and smoothness and tackiness to the touch. The results are shown in Table 2.

**Table 2**

| | Practical Example 7 and 8 | | | | Comp. Ex. 5 and 6 | | |
|---|---|---|---|---|---|---|---|
| | Emulsion obtained in: | | | | | | |
| | Pr. Ex. 1 | Pr. Ex. 2 | Pr. Ex. 3 | Pr. Ex. 4 | Comp. Ex. 1 | Comp. Ex. 3 | Comp. Ex. 4 |
| Smoothness of skin | ○ | ○ | ○ | ○ | ○ | Δ | Δ |
| Sensation of tackiness on the skin | ○ | ○ | ○ | Δ | × | Δ | Δ |
| Coefficient of adhesion to hair | 0.06% | 0.06% | 0.07% | 0.07% | 0.03% | 0.02% | 0.03% |
| Sensation of moistness of hair | ○ | ○ | ○ | ○ | Δ | × | × |
| Sensation of smoothness of hair | ○ | ○ | ○ | Δ | Δ | × | Δ |

### [Practical Example 9]

After mixing glycerol, a 1.0 wt% aqueous solution of a carboxyvinyl polymer, and water in a homogenizer, the pH of the mixture was adjusted to 6.5 by adding a 50% aqueous solution of sodium hydroxide. Three portions of the mixture were then combined with ethanol and emulsions of Practical Examples 2,5, and 6, whereby three liquid cosmetic lotions A, B, and C were obtained. The quantities of the various components (in parts by weight) are shown in Table 3.
The obtained liquid cosmetic lotions A, B, C were subjected to a functional test in which they were evaluated by a panel of 10 people with regard to tactile sensation (sliding sensation, smoothness to the touch). A 13.2 cm² circle was drawn on the inner side of the forearm of each panel member, a 20 µl sample was applied with a dispenser onto the skin within the boundaries of the circle, and the liquid was uniformly spread by a finger until disappearance of the moisture component. The results of the evaluation are shown in Table 3.
- Evaluation Criteria
   ○: 8 or more panelists evaluated the sliding sensation and smoothness to the touch as good.
   Δ: 4 to 7 panelists evaluated the sliding sensation and smoothness to the touch as good.
   × : 3 or less than 3 panelists evaluated the sliding sensation and smoothness to the touch as good.

### [Comparative Example 7]

Three liquid cosmetic lotions D, E, and F were prepared by the same method as in Practical Example 9, with the exception that in Practical Example 9 the emulsions obtained in Comparative Examples 1, 3, 4 were used instead of the emulsions of Practical Examples 2, 5, 6. The lotions were evaluated with regard to tactile sensations in use by the same method as in Practical Example 9. The results are shown in Table 3.

**Table 3**

| Components of liquid cosmetic lotion | Practical Example 9 | | | Comparative Example 7 | | |
|---|---|---|---|---|---|---|
| | A | B | C | D | E | F |
| Glycerol | 8 | 8 | 8 | 8 | 8 | 8 |
| Aqueous solution of carboxyvinyl polymer | 0.2 | 0.2 | 0.2 | 0.2 | 0.2 | 0.2 |
| Water | 68.8 | 68.8 | 68.8 | 68.8 | 68.8 | 68.8 |
| Ethanol | 3 | 3 | 3 | 3 | 3 | 3 |
| Emulsion of Practical Example 2 | 20 | - | - | - | - | - |
| Emulsion of Practical Example 5 | - | 20 | - | - | - | - |
| Emulsion of Practical Example 6 | - | - | 20 | - | - | - |
| Emulsion of Comparative Example 1 | - | - | - | 20 | - | - |
| Emulsion of Comparative Example 3 | - | - | - | - | 20 | - |
| Emulsion of Comparative Example 4 | - | - | - | - | - | 20 |
| Sodium Hydroxide | Appropriate amount | Appropriate amount | Appropriate amount | Appropriate amount | Appropriate amount | Appropriate amount |
| Results of evaluation of tactile sensations | ○ | ○ | ○ | X | Δ | Δ |

### [Practical Example 10]

After finely grinding a cationically-modified cellulose ether, it was mixed with coconut-oil fatty acid diethanolamide, whereupon the mixture was stirred and combined with a 25% aqueous solution of sodium alkylether sulfate, sodium salicylate, and water, after which the mixture was heated at 85°C for 2 hours. After cooling, the product was mixed with the emulsions obtained in Practical Examples 2 and 6, thereby preparing shampoos of two types A and B. The contents of the various components (in parts by weight) are shown in Table 4.
On the other hand, 5 g locks of hair were pretreated by washing with an aqueous solution of 1% sodium polyoxylaurylsulfate. The water was drained, the hair was dried for 12 hours at 25°C. The pretreated hair was coated with 1 cc of aforementioned previously obtained shampoos A and B, respectively, after which the hair was combed out with fingers during 1 min and rinsed with water. After draining the water, the remaining water was lightly wiped off with a kimtowel wiper, and the hair lock was dried for 12 hours at 25°C. The treated hair was evaluated for tactile sensation (smoothness and softness to the touch) by a panel of 10 people. The results are shown in Table 4.
- Evaluation Criteria
   o: 8 or more panelists recognized the sensation of smoothness and moistness, and the sensation was evaluated as good.
   Δ: 4 to 7 panelists recognized the sensation of smoothness and moistness, and the sensation was evaluated as good.
   × : 3 or less than 3 panelists recognized the sensation of smoothness and moistness, and the sensation was evaluated as good.

### [Comparative Example 8]

Three types of shampoos C, D, and E were prepared by the same method as in Practical Example 10, with the exception that emulsions obtained in Comparative Examples 1, 3, 4 were used instead of the emulsions obtained in Practical examples 2 and 6. The obtained shampoos were used for treating locks of hair by the same method as in Practical Example 10. The results of evaluation are shown in Table 4.

**Table 4**

| Shampoo composition | Practical Example 10 | | Comparative Example 8 | | |
|---|---|---|---|---|---|
| | A | B | C | D | E |
| Aqueous solution of 25% sodium alkylether sulfate | 72.0 | 72.0 | 72.0 | 72.0 | 72.0 |
| Coconut oil fatty acid diethanolamide | 2.0 | 2.0 | 2.0 | 2.0 | 2.0 |
| Cation-modifiable cellulose ether | 1.5 | 1.5 | 1.5 | 1.5 | 1.5 |
| Emulsion of Practical Example 2 | 2.0 | - | - | - | - |
| Emulsion of Practical Example 6 | - | 2.0 | - | - | - |
| Emulsion of Comparative Example 1 | - | - | 2.0 | - | - |
| Emulsion of Comparative Example 3 | - | - | - | 2.0 | - |
| Emulsion of Comparative Example 4 | - | - | - | - | 2.0 |
| Sodium salicylate | 0.2 | 0.2 | 0.2 | 0.2 | 0.2 |
| Water | 22.3 | 22.3 | 22.3 | 22.3 | 22.3 |
| Results of evaluation of tactile sensations | ○ | ○ | Δ | × | × |

### [Practical Example 11]

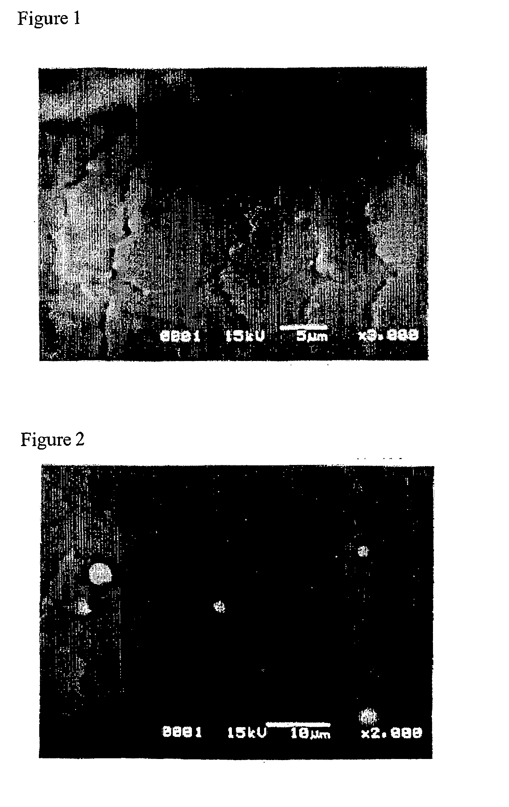

The emulsions obtained in Practical Examples 1, 2, and 6 were diluted with water until the concentration of the mixture of the organosilicon compound and oil became reached 0.2 wt%. At the same time, 5 g locks of hair were washed with aqueous solutions of 1 wt% sodium polyoxylaurylsulfate. The hair was rinsed with water and dried for 12 hours at 25°C. Subsequently, the treated hair locks were immersed for 30 sec into a 500g of an aqueous solution of the emulsions obtained as described earlier. After draining the water, the remaining water was lightly wiped off with a kimtowel wiper, and the hair locks were dried for 12 hours at 25°C. The amounts of silicone adhered to the treated hair locks were determined using the Fluorescence X-ray analyzer [Mod. 3270 from Rigaku Co., Ltd.]. The results of the measurements are shown in Table 5.
The surface condition of the hair in the hair lock treated by the immersion treatment as described above with the aqueous solution of the emulsion obtained in Practical Example 6 was observed using a Scanning Microscope/Energy Dispersion-type X-Ray Analyzer (JSM-5800 LV/JED 2100, manufactured by Nippon Denshi Co., Ltd.). The observation showed that silicone formed uniform coating without adhered grains (Fig. 1).
As can be seen from the results of the above observation and the results of tactile sensation evaluation given in Practical Example 10, the emulsion had good adherence to the hair and coated the hair surface with a uniform layer. It is understood, therefore, that the use of this product can be extremely efficient in improving the sensation of smoothness and tactile sensation of hair.

### [Comparative Example 9]

A lock of hair was treated with an aqueous solution by the method of Practical Example 11 using the emulsions obtained in Comparative Examples 1, 3, and 4. The amount of the adhered silicone was measured by the method of Practical Example 11. The results are shown in Table 5.
Furthermore, when the surface condition of the hair treated with an aqueous solution of the emulsion obtained in Comparative Example 1 was observed by the method of Claim 11, the observation revealed silicone grains adhered to the hair surface (Fig. 2). The grains reduced the amount of the adhered silicone and made the coating non-uniform. It can be appreciated, therefore, that this emulsion is not sufficiently effective for improving the tactile sensation of hair.

**Table 5**

| | Practical Example 11 | | | Comparative Example 9 | | |
|---|---|---|---|---|---|---|
| | Emulsion obtained in: | | | | | |
| | Pr. Ex. 1 | Pr. Ex. 2 | Pr. Ex. 6 | Comp. Ex. 1 | Comp. Ex.3 | Comp. Ex. 4 |
| Amount of silicone adhered to hair | 0.10 | 0.12 | 0.14 | 0.07 | 0.07 | 0.04 |

### Industrial Applicability

The aqueous emulsion of the present invention is an aqueous emulsion of a mixture of the above-described component (A) and component (B) and is characterized by superior dispersibility and adhesive properties as well as by possessing excellent lubricating properties. In addition, the production process of the present invention is characterized by the capability to efficiently produce aqueous emulsions with a small particle size containing a finely dispersed mixture of a high-molecular weight organosilicon polymer and oil. Additionally, the cosmetic materials of the present invention are characterized by imparting superior characteristics such as smoothness and moist feel to the skin and hair.

## Claims

1. An aqueous emulsion comprising as the disperse phase a mixture consisting of (A) a linear organosilicon polymer whose main chain is composed of diorganosiloxane units and alkylene units and having a number average molecular weight of 100,000 or more and (B) an oil that is liquid at room temperature and which does not contain hydrosilation-reactive groups, the weight ratio of component (A) to component (B) in said mixture being (A):(B) = 1:2 to 1:50.

2. The aqueous emulsion according to claim 1, in which the viscosity of the mixture at 25°C is not more than 1,000,000mPa s.

3. The aqueous emulsion according to claim 1, in which the number-average molecular weight of component (A) is at least 100,000.

4. The aqueous emulsion according to claim 1, in which component (B) is an isoparaffin oil or a polyorganosiloxane oil whose viscosity at 25°C is not more than 100,000mPa s.

5. A process for producing the aqueous emulsion according to claim 1, in which a mixture of 100 parts by weight of (a) a diorganopolysiloxane having silicon-bonded hydrogen atoms at the two ends of the molecular chain, 0.2 to 800 parts by weight of (b) a diolefin or diorganopolysiloxane having silicon-bonded alkenyl groups at the two ends of the molecular chain, and (B) an oil that is liquid at room temperature and which does not contain hydrosilation-reactive groups, is emulsified in water, and, in this state, component (a) and component (b) are addition-polymerized using 0.1 to 1000 ppm of (d) a hydrosilation reaction catalyst, wherein the weight ratio of the total of components (a) and (b) to component (B) in the mixture is (a) + (b): (B) = 1:2 to 1:50.

6. A cosmetic composition comprising cosmetic materials and an aqueous emulsion according to any one of claims 1 to 4.

7. A cosmetic composition according to claim 6 which is a skin cosmetic selected from hand creams, skin creams, foundations, eye shadows, face cleansers, and body shampoos.

8. A cosmetic composition according to claim 6 which is a hair cosmetic selected from shampoos, hair rinses, hair conditioners, hair treatments, setting lotions, blow-styling agents, hair sprays, styling foams, styling jels, hair liquids, hair tonics, hair creams, hair-growth stimulators, hair-nourishing preparations, and hair dye compositions.

9. Use of an aqueous emulsion according to any one of Claims 1 to 4 in cosmetic materials to impart superior characteristics such as smoothness and/or moist feel to the skin and hair.

## Patentansprüche

1. Eine wässrige Emulsion, enthaltend als die disperse Phase eine Mischung, bestehend aus (A) einem linearen Organosiliciumpolymer, dessen Hauptkette aus Diorganosiloxaneinheiten und Alkyleneinheiten gebildet ist und das ein zahlenmittleres Molekulargewicht von 100.000 oder mehr hat, und (B) einem Öl, das bei Raumtemperatur flüssig ist und keine hydrosilylierungsreaktiven Gruppen enthält, wobei das Gewichtsverhältnis der Komponente (A) zu Komponente (B) in dieser Mischung (A):(B) = 1:2 bis 1:50 ist.

2. Die wässrige Emulsion nach Anspruch 1, in der die Viskosität der Mischung bei 25°C nicht größer als 1.000.000 mPa·s ist.

3. Die wässrige Emulsion nach Anspruch 1, in der das zahlenmittlere Molekulargewicht der Komponente (A) wenigstens 100.000 ist.

4. Die wässrige Emulsion nach Anspruch 1, in der die Komponente (B) ein Isoparaffinöl oder ein Polyorganosiloxanöl ist, dessen Viskosität bei 25°C nicht größer als 100.000 mPa·s ist.

5. Ein Verfahren zur Herstellung der wässrigen Emulsion nach Anspruch 1, bei dem eine Mischung aus 100 Gewichtsteilen von (a) einem Diorganopolysiloxan mit siliciumgebundenen Wasserstoffatomen an den beiden Enden der Molekülkette, 0,2 bis 800 Gewichtsteilen von (b) einem Diolefin oder Diorganopolysiloxan mit siliciumgebundenen Alkenylgruppen an den beiden Enden der Molekülkette und (B) einem Öl, das bei Raumtemperatur flüssig ist und das keine hydrosilylierungsreaktiven Gruppen enthält, in Wasser emulgiert wird, und in diesem Zustand Komponente (a) und Komponente (b) unter Verwendung von 0,1 bis 1000 ppm (d) eines Hydrosilylierungsreaktionskatalysators, durch Addition polymerisiert werden, wobei das Gewichtsverhältnis der Summe der Komponenten (a) und (b) zu Komponente (B) in der Mischung (a) + (b) : (B) = 1:2 bis 1:50 ist.

6. Eine kosmetische Zusammensetzung, enthaltend kosmetische Stoffe und eine wässrige Emulsion nach einem der Ansprüche 1 bis 4.

7. Eine kosmetische Zusammensetzung nach Anspruch 6, welches eine Hautkosmetik ist, ausgewählt aus Handcremes, Hautcremes, Grundierungen, Lidschatten, Gesichtsreinigern und Körpershampoos.

8. Eine kosmetische Zusammensetzung nach Anspruch 6, welches eine Haarkosmetik ist, ausgewählt aus Shampoos, Haarspülungen, Haarkonditionierungsmitteln, Haarbehandlungsmitteln, Haarfestigern, Fönlotionen, Haarsprays, Stylingschäumen, Stylinggelen, Haarwässern, Haartonika, Haarcremes, Haarwuchsstimulatoren, Haarkuren und Haarfärbezusammensetzungen.

9. Verwendung einer wässrigen Emulsion nach einem der Ansprüche 1 bis 4 in kosmetischen Stoffen, um Haut und Haar überragende Charakteristika, wie z.B. Glätte und/oder ein Feuchtigkeitsgefühl zu verleihen.

## Revendications

1. Emulsion aqueuse comprenant, en tant que phase dispersée, un mélange constitué de (A) un polymère organosilicium linéaire dont la chaîne principale est composée d'unités diorganosiloxane et d'unités alkylène et qui a un poids moléculaire de nombre moyen de 100 000 ou plus et de (B) une huile qui est liquide à la température de la pièce et qui ne contient pas de groupes réactifs par hydrosilation, le rapport pondéral du composant (A) au composant (B) dans ledit mélange étant (A)/(B) = de 1/2 à 1/50.

2. Emulsion aqueuse selon la revendication 1, dans laquelle la viscosité du mélange à 25°C n'est pas supérieure à 1 000 000 mPa s.

3. Emulsion aqueuse selon la revendication 1, dans laquelle le poids moléculaire de nombre moyen du composant (A) est d'au moins 100 000.

4. Emulsion aqueuse selon la revendication 1, dans laquelle le composant (B) est une huile d'isoparaffine ou une huile de polyorganosiloxane dont la viscosité à 25°C n'est pas supérieure à 100 000 mPa s.

5. Procédé de production d'une émulsion aqueuse selon la revendication 1, dans lequel un mélange de 100 parties en poids de (a) un diorganopolysiloxane ayant des atomes d'hydrogène liés au silicium aux deux extrémités de la chaîne moléculaire, de 0,2 à 800 parties en poids de (b) une dioléfine ou un diorganopolysiloxane ayant des groupes alcényles liés au silicium aux deux extrémités de la chaîne moléculaire, et de (B) une huile qui est liquide à la température de la pièce et qui ne contient pas de groupes réactifs par hydrosilation, est émulsifié dans l'eau, et dans lequel, dans cet état, le composant (a) et le composant (b) sont polymérisés par addition en utilisant de 0,1 à 1 000 ppm de (d) un catalyseur de réaction d'hydrosilation, dans lequel le rapport pondéral du total des composants (a) et (b) au composant (B) dans le mélange est (a) + (b) / (B) = de 1/2 à 1/50.

6. Composition cosmétique comprenant des matériaux cosmétiques et une émulsion aqueuse selon l'une quelconque des revendications 1 à 4.

7. Composition cosmétique selon la revendication 6 qui est un cosmétique pour la peau choisi parmi les crèmes pour les mains, les crèmes pour la peau, les fonds de teint, des ombres à paupières, les nettoyants pour le visage, et les shampooings pour le corps.

8. Composition cosmétique selon la revendication 6 qui est un cosmétique capillaire choisi parmi les shampooings, les après-shampooings, les revitalisants capillaires, les traitements capillaires, les lotions fixantes, les agents gonflants, les laques, les mousses à coiffer, les gels à coiffer, les liquides pour les cheveux, les toniques pour les cheveux, les crèmes pour les cheveux, les stimulateurs de la croissance capillaire, les préparations nourrissantes pour les cheveux, et les compositions pour teinture capillaire.

9. Utilisation d'une émulsion aqueuse selon l'une quelconque des revendications 1 à 4 dans les matériaux cosmétiques pour conférer des caractéristiques supérieures telles que la douceur et/ou la sensation de fraîcheur à la peau et aux cheveux.
